# EUROPEAN PATENT APPLICATION

(11) **EP 3 598 973 A1**
(43) Date of publication of application: **29.01.2020**
(21) Application number: 18382555.3
(22) Date of filing: 24.07.2018
(51) Int. Cl.: A61K 31/395, C07K 16/28, A61K 39/395, A61P 35/00

(54) **COMBINED THERAPY AGAINST CANCER**

(71) Applicant: Fundación del Sector Público Estatal Centro Nacional de Investigaciones Oncológicas Carlos III (F.S.P. CNIO), 28029 Madrid (ES)
(72) Inventor: Barbacid, Mariano, Madrid (ES); Guerra, Carmen, Madrid (ES); Blasco, María Teresa, Madrid (ES); Navas, Carolina, Madrid (ES)
(74) Representative: Elzaburu S.L.P.

(57) **Abstract**

The present invention relates to a pharmaceutical composition comprising an inhibitor of the expression, activity and/or function of c-Raf and an inhibitor of the expression, activity and/or function of EGF Receptor (EGFR) and its use in the treatment of pancreatic cancer.

SEQUENCE LISTING

<110>  FUNDACIÓN DEL SECTOR PÚBLICO ESTATAL CENTRO NACIONAL DE
       INVESTIGACIONES ONCOLÓGICAS CARLOS III

<120>  COMBINED THERAPY AGAINST CANCER

<130>  EP-1859

<160>  3

<170>  PatentIn version 3.5

<210>  1
<211>  21
<212>  DNA
<213>  Mus musculus

<400>  1
ctgattgccc aactgccata a                                                21

<210>  2
<211>  23
<212>  DNA
<213>  Mus musculus

<400>  2
gagtcagcaa atgcactgaa atg                                              23

<210>  3
<211>  21
<212>  DNA
<213>  Mus musculus

<400>  3
actgatctgg agcacagcaa t                                                21

## Description

### FIELD OF THE INVENTION

The present invention relates to a combined therapy against cancer which has proven to be particularly useful in the prevention and treatment of pancreatic cancer.

### BACKGROUND

Pancreatic ductal adenocarcinoma (PDAC) is the third cause of cancer deaths in the US and is projected to be second after non-small cell lung cancer (NSCLC) by 2030 (Rahib et al., 2014)¹. The 5-year survival remains around 5% due to the lack of new effective treatments. Indeed, gemcitabine, approved back in 1997 is still the standard of care^{2,3}. Combination of gemcitabine with nab-paclitaxel or erlotinib has shown rather modest improvements^{4,5}. Other therapies such as FOLFIRINOX are rather toxic and can only be administered to selected patients⁶. The main genetic drivers of PDAC have been identified⁷. Whereas activating mutations in K-RAS are believed to be the main initiating event, additional mutations in several tumor suppressors including TP53, CDKN2A, SAMD4, BRCA2 and TGFβR contribute to tumor progression. So far, genetic silencing of the initiating K-Ras oncogenic in a GEM tumor model has been the only "therapeutic strategy" capable of inducing tumor regression^{8,9}. Unfortunately, K-RAS oncogenes remain undruggable in spite of promising efforts¹⁰. On the other hand, K-RAS oncogenic signaling is mediated by two signaling cascades made up of druggable kinases¹¹. Previous studies have shown that ablation of the EGF receptor (EGFR) delayed PDAC development in K-Ras/Tp53 driven GEM tumor models^{12,13}. More recently, we have identified c-Raf as a suitable therapeutic target for K-Ras/Tp53 driven lung adenocarcinoma¹⁴. Here we describe that combined, systemic ablation of EGFR and c-Raf expression in advanced K-Ras/Tp53 mutant PDAC tumors results in complete regression of a significant percentage of tumors with tolerable toxicities. These observations may open the door to the development of rational targeted therapies for PDAC tumor patients.

WO2015087279 A1 describes the triple combination of a B-RAF inhibitor (dabrafenib), a MEK inhibitor (trametinib) and a EGFR inhibitor (panitumumab) and its use in the treatment of cancer. It has been described that the inhibition of B-Raf is not useful because it inhibits the complete pathway and its toxicity is quite high (Blasco et al 2011 Cancer Cell 19, 652-663).

Inhibiting K-Ras and MEK also implies intolerable side effects due to the toxicity of the treatment.

### DESCRIPTION OF THE INVENTION

The simultaneous deletion of Egfr and c-Raf in PDAC results in a significant therapeutic effect with an extremely low toxicity.

The present invention represents a very promising therapy in the treatment of pancreatic cancer since it leads to a complete tumor regression while its side effects are much smaller than other therapies described up to date.

In a first aspect, the present invention relates to a pharmaceutical composition comprising an inhibitor of the expression, activity and/or function of c-Raf and an inhibitor of the expression, activity and/or function of EGF Receptor (EGFR).

In a preferred embodiment, the inhibitor of the expression, activity and/or function of c-Raf comprises a c-Raf inhibitor compound, a c-Raf inhibitor antibody or an antigen binding fragment thereof, a peptide or a nucleotide sequence.

In a preferred embodiment, the inhibitor of the expression, activity and/or function of c-Raf does not inhibit C-RAF kinase activity.

In a preferred embodiment, the inhibitor of the expression, activity and/or function of c-Raf is other than sorafenib, vemurafenib, dabrafenib y LY3009120.

In a preferred embodiment, the inhibitor of the expression, activity and/or function of c-Raf does not inhibit the expression, activity and/or function of b-Raf.

In a preferred embodiment, the inhibitor of the expression, activity and/or function of EGFR comprises an EGFR inhibitor compound, an EGFR inhibitor antibody or an antigen binding fragment thereof, a peptide or a nucleotide sequence.

In a preferred embodiment, the nucleotide sequence is or codifies for a guide RNA, an interfering RNA or a micro RNA.

In a preferred embodiment, the inhibitor of the expression, activity and/or function of EGFR is selected from afatinib, erlotinib, gefitinib, brigatinib, icotinib, neratinib, lapatinib, vandetanib, osimertinib, cetuximab, panitumumab, necitumumab, nimotuzumab, zalutumumab, matuzumab and combinations thereof.

In a preferred embodiment, the pharmaceutical composition comprises a therapeutically effective amount of an inhibitor of the expression, activity and/or function of c-Raf, a therapeutically effective amount of an inhibitor of the expression, activity and/or function of EGFR and optionally a pharmaceutically acceptable excipient.

In another aspect, the present invention relates to the pharmaceutical composition according to any one of the preceding claims for use in the prevention and/or treatment of cancer. Preferably, for use in the prevention and/or treatment of pancreatic cancer. More preferably, for use in the prevention and/or treatment of pancreatic intraepithelial neoplasia or of pancreatic ductal adenocarcinoma. In a preferred embodiment, the pharmaceutical composition is for use in tumor regression in a subject afflicted with cancer. Preferably, for use in tumor regression in a subject afflicted with pancreatic cancer. More preferably, for use in tumor regression in a subject afflicted with pancreatic intraepithelial neoplasia or of pancreatic ductal adenocarcinoma.

An aspect of the present invention is a method for the prevention and/or treatment of cancer comprising the administration of a therapeutically effective amount of an inhibitor of the expression, activity and/or function of c-Raf and a therapeutically effective amount of an inhibitor of the expression, activity and/or function of EGF Receptor (EGFR). Preferably, the method of the present invention is for the treatment and/or prevention of pancreatic cancer, more preferably of pancreatic intraepithelial neoplasia (PanIN) or of PDAC.

### DESCRIPTION OF THE DRAWINGS

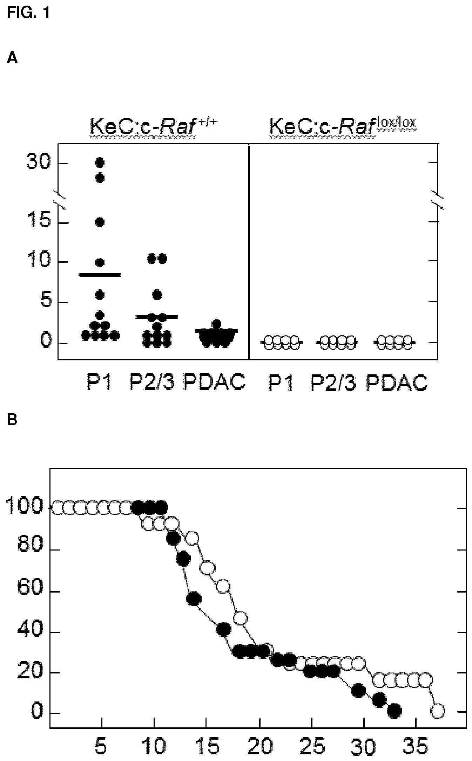

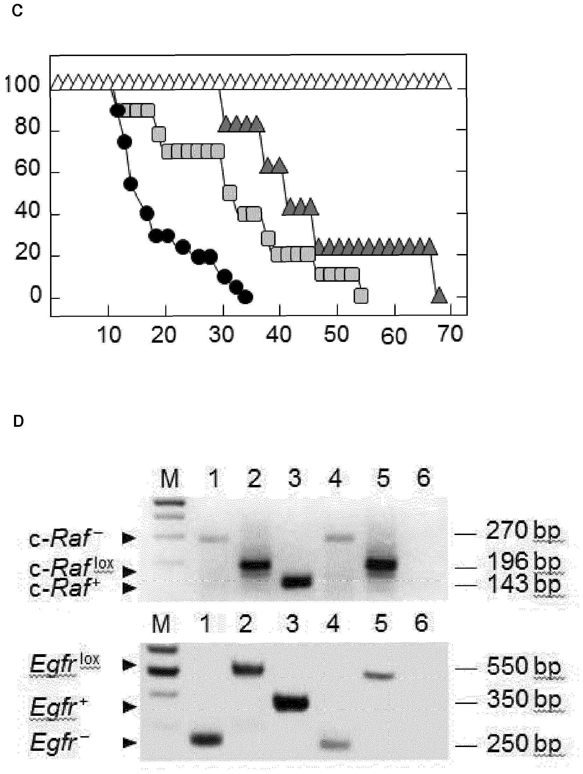

**Figure 1****.** Effect of *c-Raf* and *Egfr* ablation in initiation of K-Ras driven pancreatic lesions. **a,** Number of low- and high-grade PanIN lesions and PDAC tumors in one year old KeC mice carrying either wild type c-*Raf*⁺ (solid circles, n=12) or conditional c-*Raf*^{lox} (open circles, n=8) alleles. Horizontal bars indicate the average number of lesions per mouse. **b,** Survival of KPeC;c-*Raf*^{+/+} (solid circles, n=20) and KPeC;c-*Raf*^{lox/lox} (open circles, n=13) mice. **c,** Survival of KPeC;c-*Raf*^{+/+} (solid circles, n=20), KPeC;c-Raf ^{+/lox};*Egfr*^{lox/lox} (grey squares, n=10), KPeC;c-*Raf*^{lox/lox};*Egf*^{+/lox} (grey triangles, n=5) and KPeC;c-*Raf*^{lox/lox};*Egfr*^{lox/lox} (open triangles, n=14) mice. **d,** PCR analysis of *c-Raf* and *Egfr* alleles using DNA extracted from K-Ras^{G12V} expressing, X-Gal positive acinar cells isolated by Laser Capture Microdissection. Migration of c*-Raf* and *Egfr⁻ null* alleles (lane 1) *c-Raf*^{lox and} *Egfr*^{lox} conditional floxed alleles (lane 2) and *c-Raf*^{+and} *Egfr*⁺ wild type alleles (lane 3). DNA extracted from X-Gal positive (lane 4) and X-Gal negative (lane 5) acinar cells of KPeC;c-*Raf*^{lox/lox};*Egfr*^{lox/lox mice.} Lane 6 is a blank control. Lane M depicts a DNA ladder. Expected size for each DNA fragment is indicated.

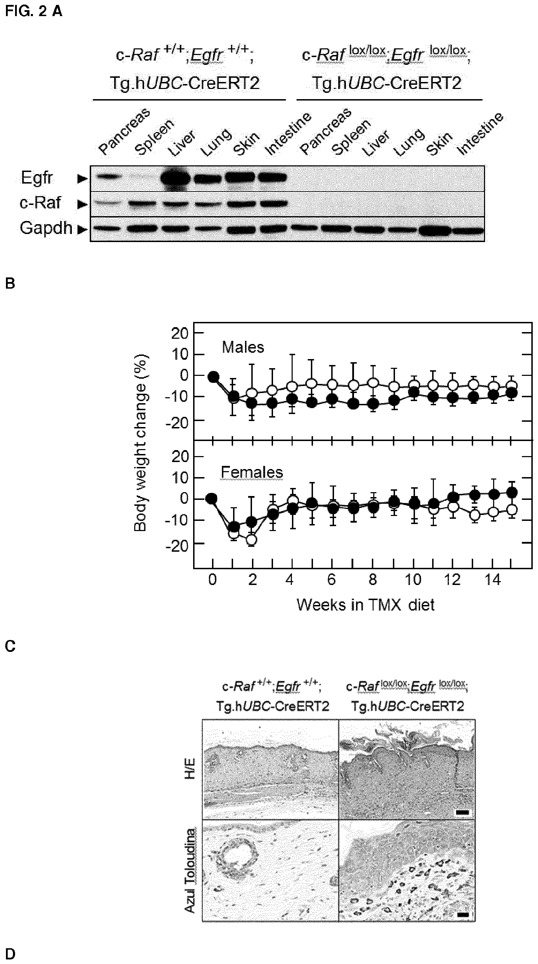

**Figure 2****.** Ablation of c-Raf and Egfr expression induces acceptable toxicities. **a**, Western blot analysis illustrating the expression of c-Raf and Egfr in the indicated tissues of c-*Raf*^{+/+};*Egfr*^{+/+};Tg.h*UBC*-CreERT2^{+/T} and c-*Raf*^{lox/lox};*Egfr*^{lox/lox};Tg.h*UBC-*CreERT2^{+/T} mice after 3 weeks of TMX exposure. Gapdh expression served as a loading control. Arrowheads show migration of the indicated proteins. **b**, Body weight change in grams (g) of male (upper) and female (lower) *c-Raf*^{+/+};*Egfr*^{+/+};Tg.h*UBC-*CreERT2^{+/T} (solid circles, n=5) and c-*Raf*^{lox/lox};*Egfr*^{lox/lox};Tg.h*UBC*-CreERT2^{+/T} mice (open circles n=5) exposed to TMX for the indicated length of time. **c**, Representative H&E and Toluidine-blue staining of skin sections from c-*Raf*^{+/+};*Egfr*^{+/+};Tg.h*UBC*-CreERT2^{+/T} and c-*Raf*^{lox/lox};*Egfr*^{lox/lox};Tg.h*UBC*-CreERT2^{+/T} mice exposed to TMX for 15 weeks. Scale bars represent 100 µm (H&E) and 20 µm (toluidine blue). **d**, Representative H&E and cleaved Caspase-3 staining in sections of small intestine of c*-Raf*^{+/+};*Egfr*^{+/+};Tg.h*UBC-*CreERT2^{+/T} and c-*Raf*^{lox/lox};*Egfr*^{lox/lox};Tg.h*UBC*-CreERT2^{+/T} mice exposed to TMX for 15 weeks. Scale bars represent 100 µm (H&E) and 20 µm (cleaved Caspase-3).

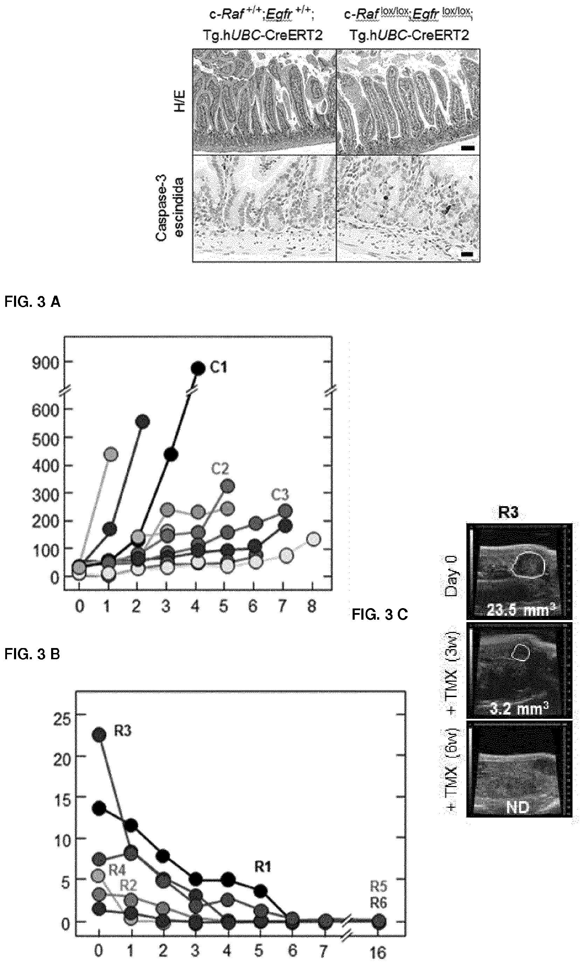

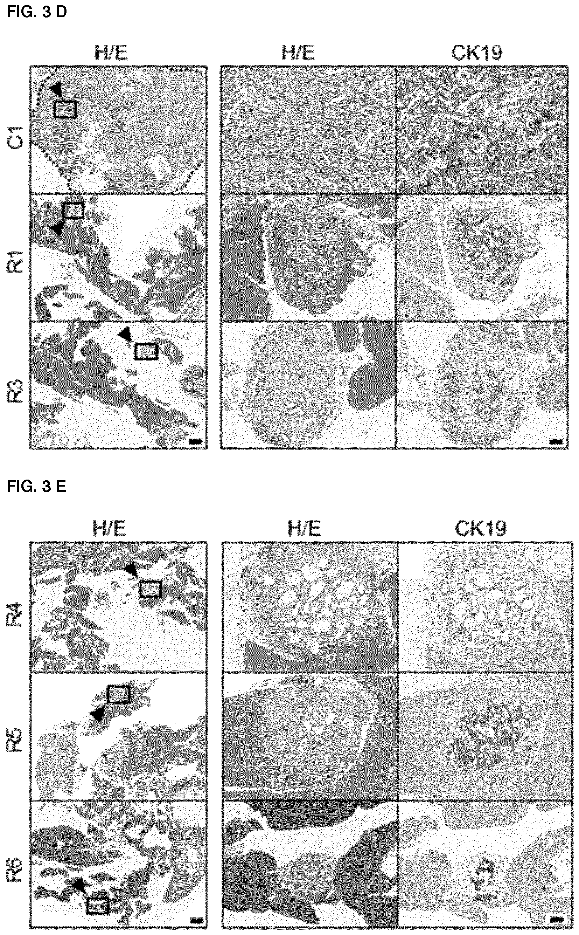

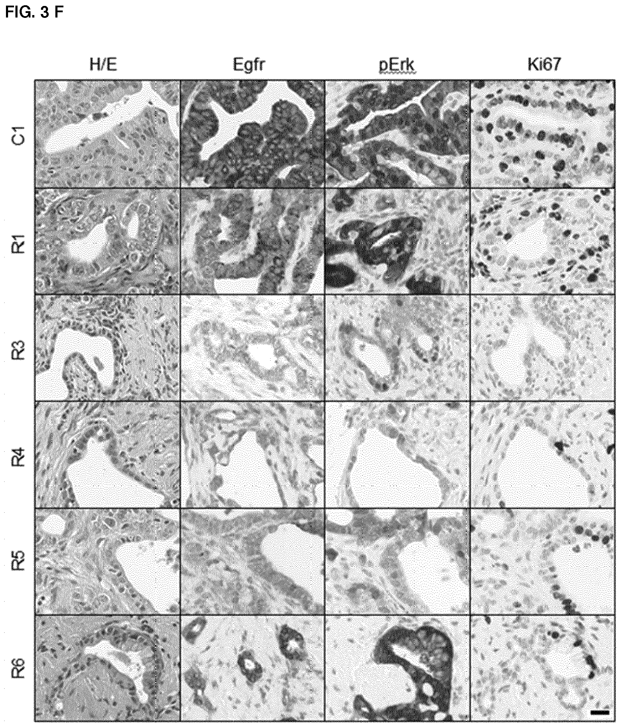

**Figure 3****.** *c-Raf* and *Egfr* ablation induce regression of a fraction of PDAC tumors. **a**, Total tumor volume visualized by weakly ultrasound monitorization of KPeFC; c-*Raf*^{+/+};*Egfr*^{+/+} control mice (n=10 mice, 15 tumors) exposed to a TMX diet for the indicated time. Each color represents a different mouse. **b**, Total tumor volume visualized by weakly ultrasound monitorization of KPeFC; *c-Rat*^{lox/lox}*;Egf*^{lox/lox} mice (n= 6 mice, 7 tumors) exposed to a TMX diet for the indicated time. Each color represents a different mouse (R1-R6). **c**, Representative ultrasound images of the regression of a large tumor (23.5 mm³) present in the R3 mouse after 3 and 6 weeks of TMX exposure. Visible lesions are outlined. Tumor volumes are indicated. ND: not detectable. **d,** (left column) Representative H&E stained paraffin sections of the pancreata of control KPeFC;c-*Raf*^{+/+};*Egfr*^{+/+} C1 ^{and} KPeFC;c-*Raf*^{lox/lox};*Egfr*^{lox/lox} R1 and R3 mice after six weeks of TMX exposure. The tumor present in the C1 mouse is outlined by a dotted line. Scale bar represents 1000 µm. Box insets, indicated by arrowheads, mark areas shown at higher magnification in the images shown to the right stained with H&E (H/E) and Cytokeratin19 (CK19). Scale bar represents 100 µm. **e**, Same as in **d** for tumors present in KPeFC;c-*Raf*^{lox/lox};*Egfr*^{lox/lox} R4-R6 mice. **f**, Higher magnification of representative H&E, Egfr, pErk and Ki67 stained paraffin sections from KPeFC;c-*Raf*^{+/+};*Egfr*^{+/+(C1)and} KPeFC;c-*Raf*^{lox/lox};*Egfr*^{lox/lox} (R1, R3-R6) mice. Scale bar represents 20 µm. The R2 mouse did not had a scar lesions.

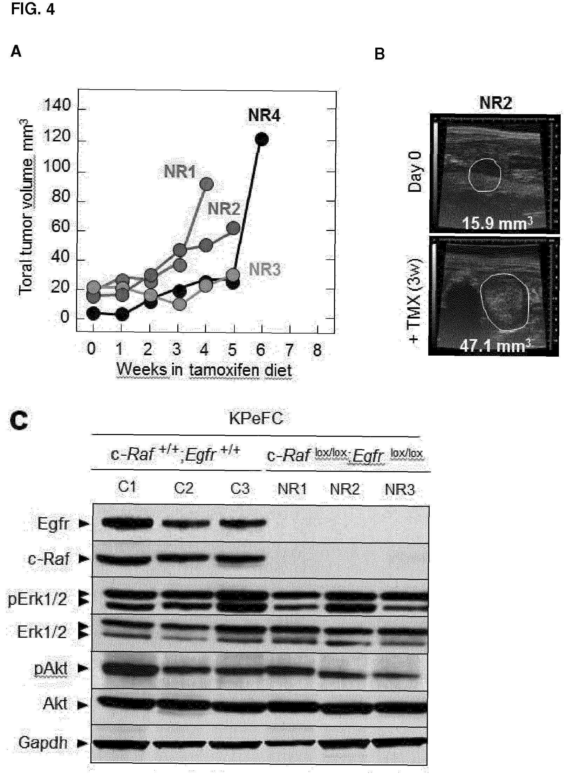

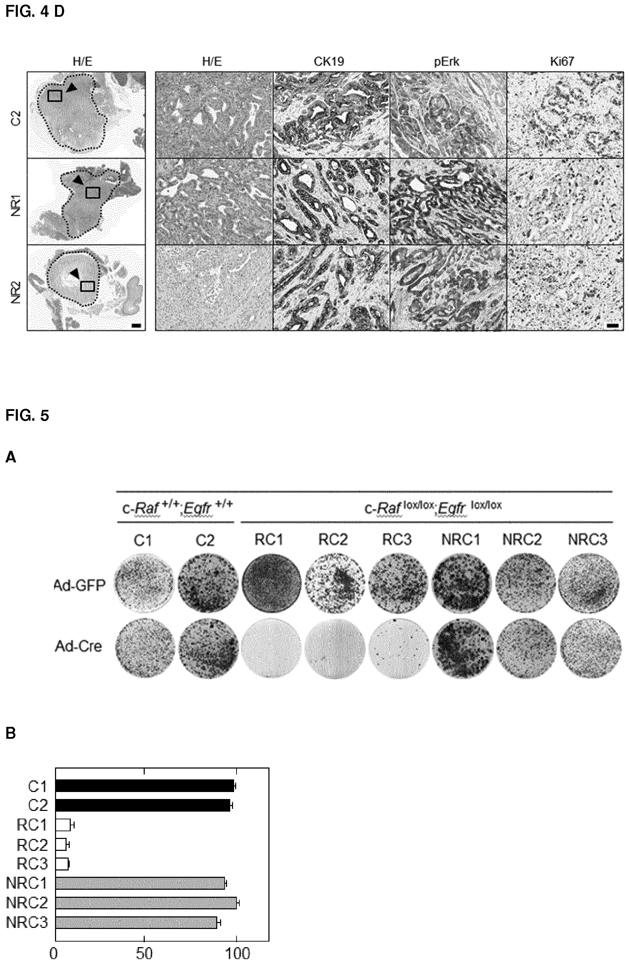

**Figure 4****.** A fraction of PDAC tumors do not respond to *c-Raf* and *Egfr* ablation. **a**, Total umor volume visualized by weakly ultrasound monitorization of KPeFC;c-*Raf*^{lox/lox};*Egfr*^{lox/lox} mice (n=4 mice, 4 tumors) exposed to a TMX diet for the indicated time. Each color represents a different mouse (NR1-NR4). **b**, Representative ultrasound images of the progression of the tumor present in the NR2 mouse after 3 weeks of TMX exposure. Visible lesions are outlined in white. Tumor volumes are indicated. **c**, Western blot analysis of c-Raf and Egfr expression in lysates obtained from PDAC tumors present in control KPeFC;c-*Raf*^{+/+};*Egfr*^{+/+} mice depicted in Figure **3a** (C1-C3) and in NR1-NR3 KPeFC;c-*Raf*^{lox/lox};*Egfr*^{lox/lox} mice exposed to TMX for six weeks. Expression levels of Erk1/2 pErk1/2, Akt and pAkt are also shown. Gapdh served as a loading control. **d**, (**left column**) Representative H&E stained paraffin sections of the pancreata of control C2 KPeFC;c-*Raf*^{+/+};*Egfr*^{+/+} mouse ⁽depicted in Fig **3a**⁾ and of non-responder NR1 and NR2 KPeFC;c-*Raf*^{lox/lox};*Egfr*^{lox/lox} mice after six weeks of TMX exposure. Tumors are outlined by dotted lines. Box insets, indicated by arrowheads, mark areas shown at higher magnification in the adjacent images shown to the right stained with H&E, Cytokeratin19 (CK19), pErk and Ki67. Scale bar represents 50 µm.

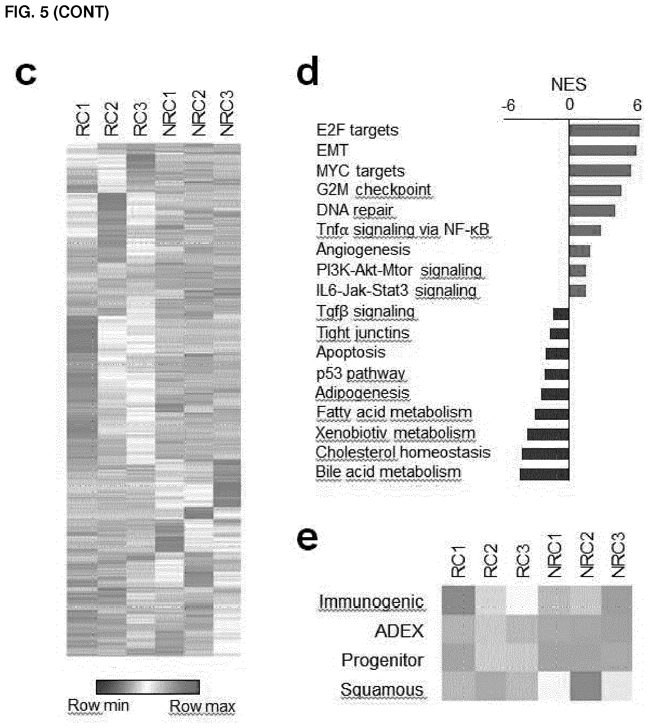

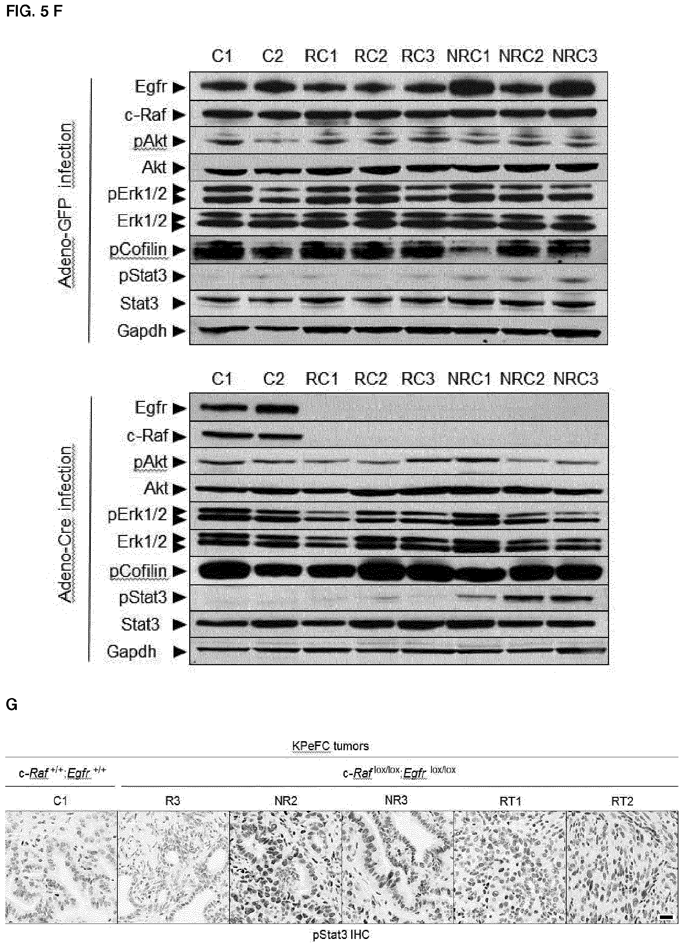

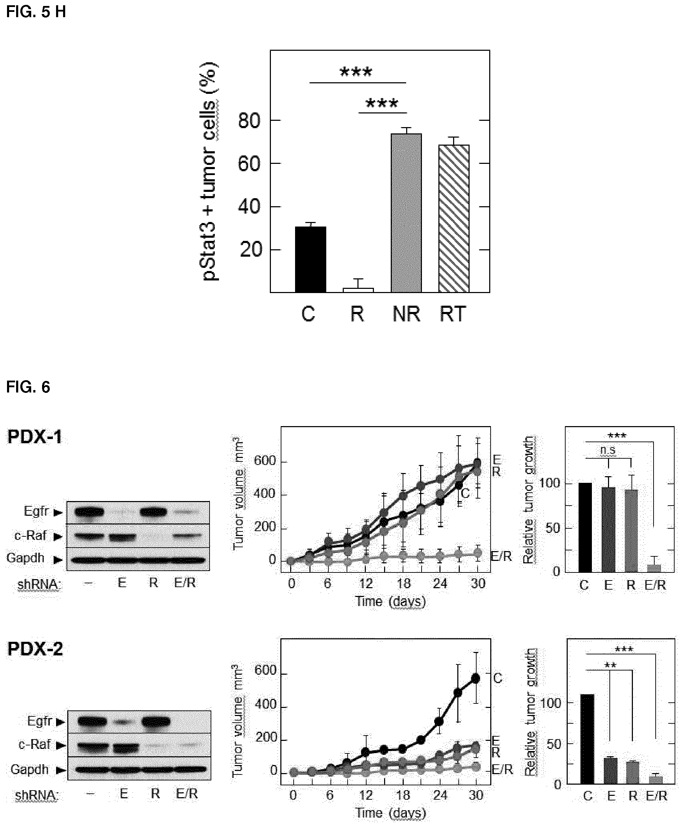

**Figure 5****.** Differential RNA expression profiles of PDAC tumor cells sensitive and resistant to *c-Raf* and *Egfr* ablation. **a**, Colony formation assay of tumor cell lines established from individual tumors of two KPeFC;c-*Raf*^{+/+};*Egfr*^{+/+} control mice (C1 and C2) and of six KPeFC;c-*Raf*^{lox/lox};*Egfr*^{lox/lox} animals These tumor cell lines are designated as "Responder cells" (RC1-RC3) or Non responder cells (NRC1-NRC3) based on their proliferative properties after ablation of c-Raf and Egfr expression with Ad-Cre particles. Infection with Ad-GFP particles was used as a negative control. Cells were fixed with glutaraldehyde and stained with crystal violet. **b**, Quantification of the number of colonies expressed as percentage of the number of colonies observed in cells infected with Ad-Cre and those infected with Ad-GFP particles. Error bars indicate standard deviation. **c**, Heat map representing color-coded expression levels of differentially expressed genes in NRC vs. RC cell lines infected with Ad-GFP particles. d, GSEA pathway analysis of NRC vs. RC cell lines infected with Ad-GFP particles. The normalized enrichment score (NES) ranking was generated by the GSEA. e, Heat map comparing the transcriptional profiles of RC and NRC cells with those of PDAC tumors as reported by Bailey and cols. **f**, Western blot analysis of Egfr, c-Raf, pAkt, Akt, pErk1/2, Erk1/2, pCofilin, pStat3 and Stat3 protein expression in whole cell extracts of the indicated cell lines obtained 5 days after (**top**) Adeno-GFP or (**bottom**) Adeno-Cre infection. Gapdh served as a loading control. **g**, pStat3 staining in paraffin-embedded sections of PDAC tumors of KPeFC;c-*Raf*^{+/+};*Egfr*^{+/+} C1 mouse and KPeFC;c-*Raf*^{lox/lox};*Egfr*^{lox/lox mice} that harbored tumors that regressed (R3), progressed (NR1, NR3) or relapsed (RT1, RT2) upon c-Raf and *Egfr* ablation. Scale bar represents 20 µm. **h**, Quantification of pStat3 positive tumor cells in paraffin-embedded PDAC tumor sections from KPeFC;c-*Raf*^{+/+};*Egfr*^{+/+} control mice (C, solid bar, n=3), KPeFC;c-*Raf*^{lox/lox};*Egfr*^{lox/lox} mice that responded to *c-Raf* and *Egfr* ablation (R, open bar, n=3). KPeFC;c-*Raf*^{lox/lox};*Egfr*^{lox/lox} mice that did not respond to *c-Raf* and *Egfr* ablation (NR, grey bar, n=3) and KPeFC;c-*Raf*^{lox/lox};*Egfr*^{lox/lox} mice that relapsed after their initial response to *c-Raf* and *Egfr* ablation (RT, striped bar, n=2). Error bars indicate standard deviation. P-values were calculated using the unpaired Student's t test. ***p < 0.001.

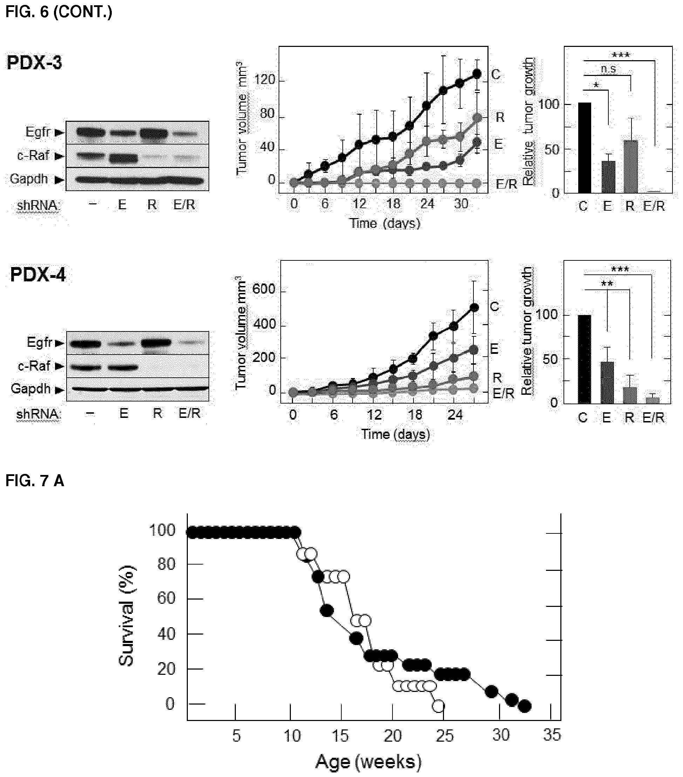

**Figure 6****.** c-Raf and Egfr expression are required for proliferation of patient-derived PDAC xenografts. **Left,** Western blot analysis of Egfr and c-Raf protein expression in whole cell extracts obtained from the indicated PDX cell line (PDX1-4) expressing a scramble shRNA (-) as well as shRNAs against *Egfr*(E), *c-Raf* (R) and *Egfr* + *c-Raf* (E/R). Gapdh served as a loading control. **Center,** Tumor growth after subcutaneous implantation in nude mice (n=4) of the corresponding PDX cell lines expressing a scramble shRNA (black) as well as shRNAs against *Egfr* (blue), *c-Raf* (red) and *Egfr* + *c-Raf* (green). **Right,** Quantification of tumor growth at the end of the experiment. Error bars indicate standard deviation. P-values were calculated using the unpaired Student's t test. *p < 0.05, **p < 0.01, ***p < 0.001. n.s: no significant.

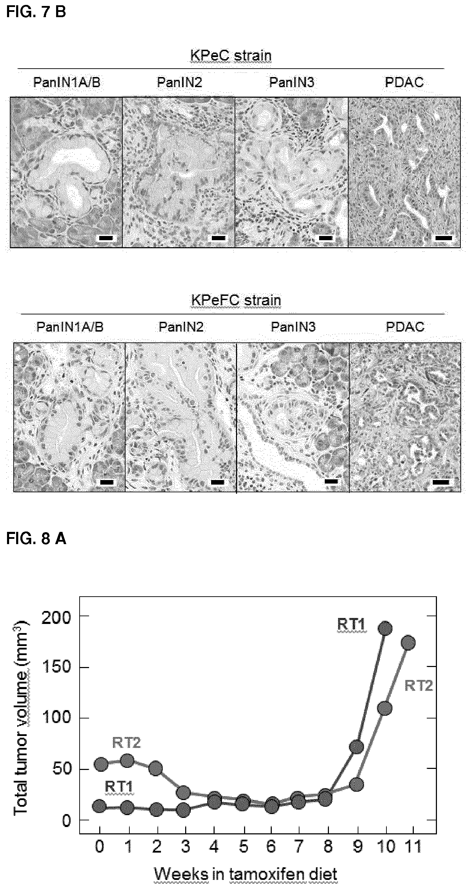

**Figure 7****.** Comparative analysis of the KPeC and KPeFC tumor models. **a**, Survival of KPeC (solid circles, n=20) and KPeFC (open circles, n=8) mice. **b**, Representative pancreatic lesions of (**top**) KPeC and (**bottom**) KPeFC mice stained with H&E. Scale bars represent 20 µm (PanlN1A/B, PanlN2, PanlN3) and 50 µm (PDAC).

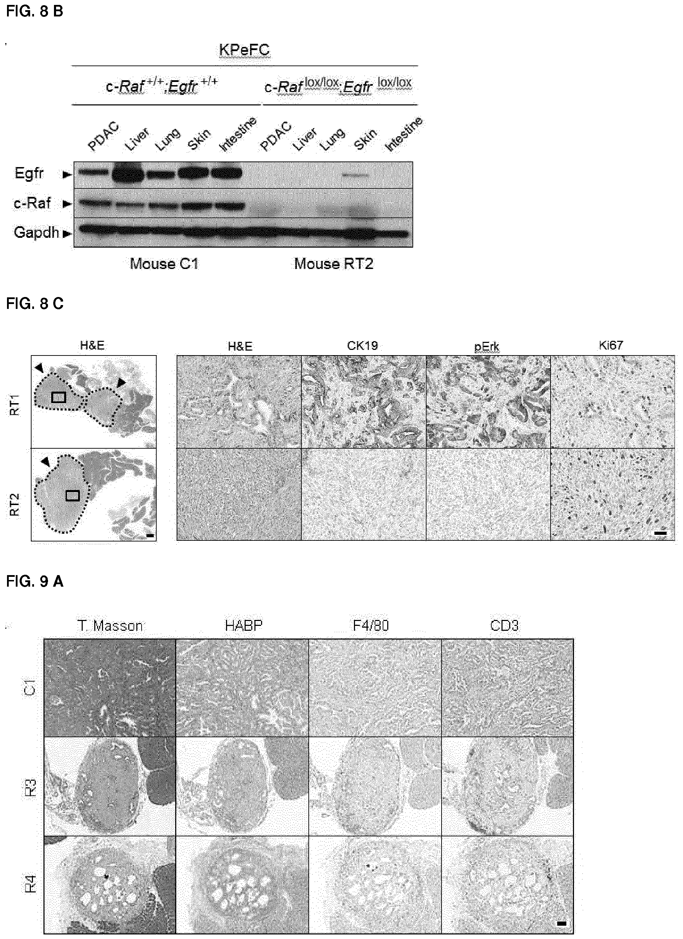

**Figure 8****.** PDAC tumors of KPeFC mice become resistant to *c-Raf* and *Egfr* ablation. **a**, Total tumor volume visualized by weakly ultrasound monitorization of KPeFC;c-*Raf*^{lox/lox};*Egfr*^{lox/lox} mice exposed to TMX. Each color represents a different mouse. **b**, Western blot analysis of c-Raf and Egfr expression in lysates derived from the PDAC tumors present in KPeFC;c-*Raf*^{+/+};*Egfr*^{+/+} control C1 mouse depicted in Figure **3a** and KPeFC;c-*Raf*^{lox/lox};*Egfr*^{lox/lox} RT2 mouse exposed to TMX for 11 weeks. Gapdh served as a loading control. **c**, (**left column**) Representative H&E stained paraffin sections of the pancreata of KPeFC;c-*Raf*^{lox/lox};*Egfr*^{lox/lox} RT1 and RT2 mice after 10 and 11 weeks of TMX exposure, respectively. Tumors are outlined by a dotted line. Scale bar represents 1000 µm. Box insets, indicated by arrowheads, mark areas shown at higher magnification in the adjacent images to the right stained with H&E, CK19, pErk and Ki67. Scale bar represents 50 µm.

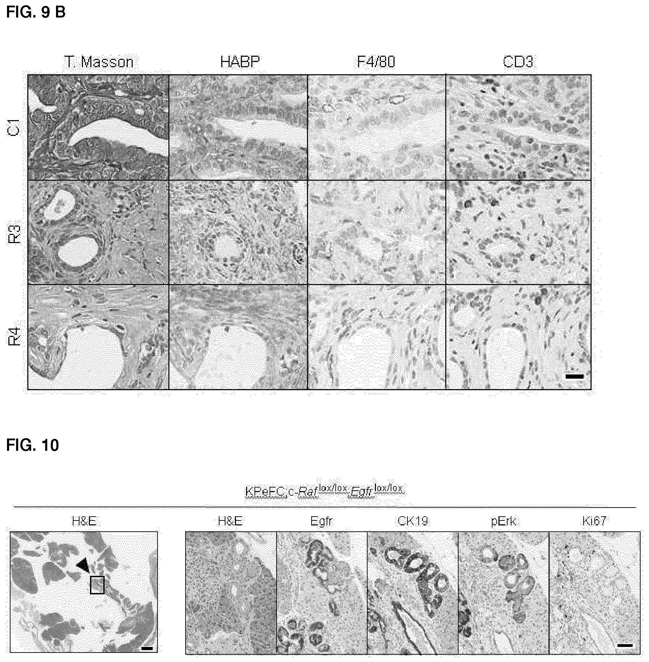

**Figure 9****.** Histological characterization of the residual scar lesions present in "Responder" mice after TMX exposure. **a**, Low and **b,** high magnification of representative sections of a PDAC tumor present in control KPeFC;c-*Raf*^{+/+};*Egfr*^{+/+} C1 mouse and in the scar lesions of KPeFC;c-*Raf*^{lox/lox};*Egfr*^{lox/lox} R3 and R4 mice stained with Masson's trichrome (T. Masson), Hyaluronic Acid Binding Protein (HABP), F4/80 and CD3. Scale bars represents **a.** 100 µm and **b,** 20 µm.
**Figure 10****.** PanIN lesions present in "Responder" mice. Representative H&E stained paraffin sections of the pancreata of KPeFC;c-*Raf*^{lox/lox};*Egfr*^{lox/lox} R2 mouse after six weeks of TMX exposure. Scale bar represents 1000 µm. Box inset, indicated by an arrowhead, marks the area shown at higher magnification in the adjacent images to the right stained with H&E, Egfr, CK19, pErk and Ki67. Scale bar represents 50 µm.

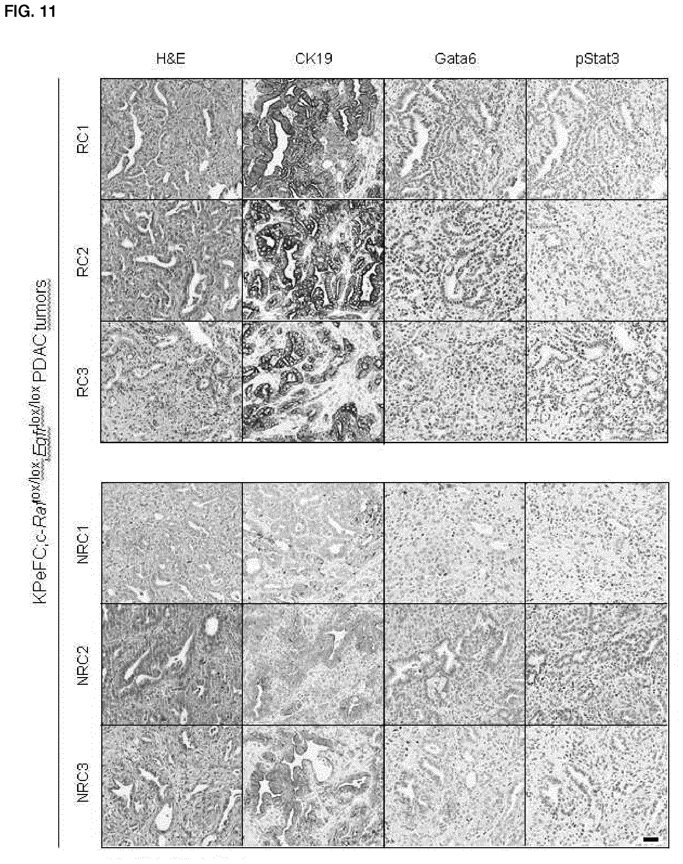

**Figure 11****.** Differential expression of CK19 and Gata6 in PDAC tumors used to generate the "Responder" and Non Responder" cell lines. Representative H&E, CK-19, Gata6 and pStat3 stained paraffin embedded sections of the original KPeFC;c-*Raf*^{lox/lox};*Egfr*^{lox/lox} tumors used to generate the (**Top**) "Responder" (RC1-RC3) and (**Bottom**) "Non responder" (NRC1-NRC3) tumor cell lines as determined by their response to *c-Raf* and *Egfr* ablation. Scale bar represents 50 µm.

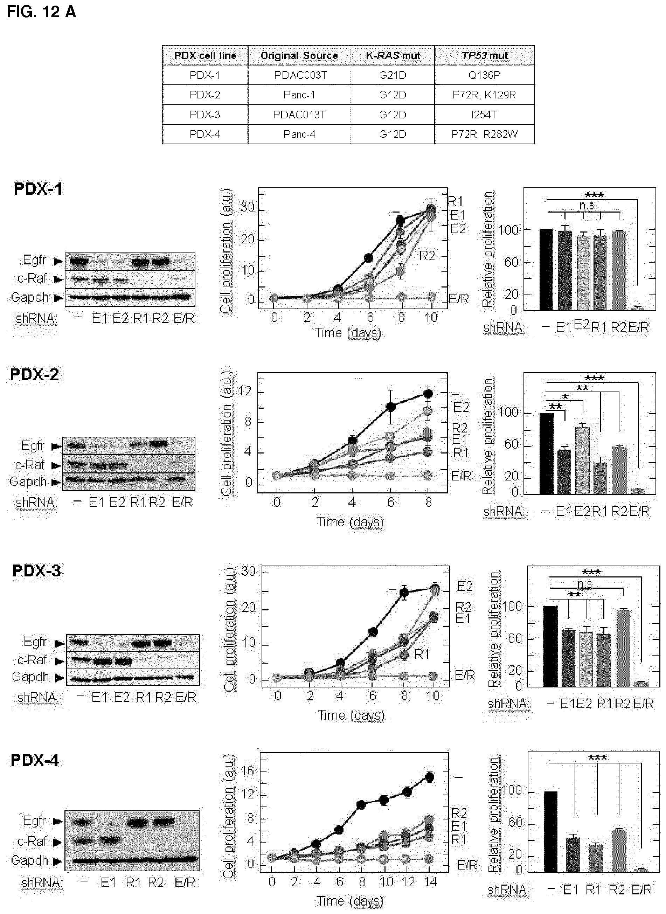

**Figure 12****.** c-Raf and Egfr are essential for proliferation of PDX cell lines *in vitro.* **a,** Original designation and *K-RAS* and *TP53* mutations of the PDX tumors used in this study. **b**, **Left,** Western blot analysis of Egfr and c-Raf expression in whole cell extracts obtained from the corresponding PDX cell line (PDX1-4) using a scramble shRNA (-), two shRNA against *Egfr* (E1, E2), two shRNAs against c-Raf (R1, R2) and the combination of shRNAs against *Egfr* and *c-Raf* (E/R). Gapdh served as loading control. **Center,** Cell proliferation assays of the corresponding PDX cell line expressing sceamble shRNA (black), shRNAs against *Egfr,* shRNAs against c-Raf and the combination of shRNAs against *Egfr* and c-*Raf*. Proliferation was determined by MTT and is expressed as arbitrary units (a.u.). Error bars indicate standard deviation. **Right,** Quantification of cell proliferation at the end of the experiment. Error bars indicate standard deviation. P-values were calculated using the unpaired Student's t test. *p < 0.05, **p < 0.01, ***p < 0.001. n.s: no significant.

### EXAMPLES

### Induction of pancreatic lesions by K-Ras^{G12V} requires c-Raf expression.

We have previously shown that c-Raf is essential for initiation and progression of K-Ras^{G12V} driven lung adenocarcinomas^{14,15}. To investigate the contribution of c-Raf to K-Ras^{G12V} driven pancreatic tumors, we added conditional c-Raf floxed alleles to the K-*Ras*^{+/LSLG12Vgeo};*Elas*-tTA/*Tet*O-Cre strain, a GEM tumor model that induces expression of K-*Ras*^{G12V} in acinar cells during late embryonic development. These mice, designated as KeC, developed PanlNs lesions with complete penetrance as well as PDAC tumors in about 20% of the animals at one year of age¹⁶. As illustrated in Fig. 1a, elimination of c-Raf alleles completely prevented PanlN formation as well as PDAC tumors. These results illustrate that c-Raf is essential for acinar cell transformation by K-Ras oncogenes.

### Ablation of Tp53 overcomes the requirement for c-Raf expression in K-Ras^{G12V} driven PanlN and PDAC lesions

Development of aggressive K-Ras driven PDAC tumors requires additional oncogenic insults such as deletion or inactivation of the Tp53 or p16/p19 tumor suppressors¹⁷⁻²⁰. To examine the effect of abrogating c-Raf signaling in the absence of Tp53, we added the floxed c*-Raf* alleles to the K-*Ras*^{+/LSLG12Vgeo};*Tp53*^{lox/lox};*Elas*-tTA/*TetO*-Cre strain (from now on KPeC mice). As illustrated in Figure 1b, deletion of c*-Raf* had no effect on the development of K-Ras/Tp53-driven PDAC tumors. All animals, regardless of whether they carried wild-type (n=20) or floxed (n=13) c-Raf alleles succumbed to pancreatic tumors around 30 weeks of age (Fig. 1b). Efficient deletion of c-Raf alleles was verified by PCR analysis of PDAC cells isolated from tumor specimens. Therefore, ablation of Tp53 completely subverted K-Ras oncogenic signals to make them independent of c-Raf. Alternatively, the absence of Tp53 may activate additional oncogenic pathways that cooperate with K-Ras^{G12V} signaling in a c-Raf independent manner.

### Concomitant ablation of c-Raf and Egfr abrogates PanlN and PDAC development in the absence of Tp53

We and others have reported that K-Ras oncogenic signals require expression of Egfr to induce PanlN lesions and PDAC tumors^{12,13}. Yet, in the absence of Tp53, ablation of Egfr expression delayed, but did not impair PDAC development^{12,13}. Therefore, we interrogated whether in the absence of Tp53, c-Raf and Egfr may signal through compensatory pathways by adding conditional c-*Raf*^{lox} and *Egfr*^{lox} alleles to KPeC mice. Control KPeC animals (n=20) succumbed to the disease with an average survival of 15 weeks of age. As expected, ablation of *Egfr* alleles increased their median survival by 80% ^{12,13}. Further elimination of a single c-*Raf*^{lox} allele (n=10) extended their median survival by almost 3-fold. Likewise, elimination of one *Egfr*^{lox} allele (n=5) doubled the median survival of KPeC mice carrying c-*Raf*^{lox} alleles. More importantly, double homozygous animals devoided of c-Raf and Egfr expression in their K-Ras^{G12V} expressing acinar cells (n=14) completely failed to produce pancreatic lesions including low and high grade PanlNs as well as overt PDAC tumors, even at one year of age. These mice retained K-Ras^{G12V} expression in their acinar cell compartment as determined by the presence of β-galactosidase, a surrogate marker for K-Ras^{G12V} expression in KPeC mice. Isolation of these cells by laser-capture microdissection confirmed the efficient recombination of c-*Raf*^{lox} and *Egfr*^{lox} alleles (Fig. 1d). No such recombination was observed in adjacent acinar cells negative for β-galactosidase expression (Fig. 1d). These results indicate that in the absence of Tp53, K-Ras^{G12V} oncogenic signaling is mediated by compensatory pathways that require expression of c-Raf and Egfr.

### Mutational complexity of PDAC tumors driven by K-Ras/Tp53 mutations.

GEM tumor models of PDAC driven by K-*Ras*^{G12V} and loss of *Tp53* closely reproduce the histopathology of human tumors^{19,20}. Yet, their mutational complexity appears to be more limited^{22,23}. Indeed, exomic next generation sequencing (NGS) of KPeC tumors revealed a similar number of miscoding mutations (13.6 mutations/tumor) (Table 1). Interestingly, none of the mutated genes identified in this genomic analysis appeared in different tumors (Table 1). Bioinformatic analysis revealed that almost half of these mutated genes felt within the twelve signaling pathways identified in human PDAC tumors²³.

**Table 1. Genes mutated in PDAC tumors of KPeCmice analyzed by exomic NGS sequencing.**

| **KPeC tumor** | **No. of mutations** | **Mutated Genes** |
|---|---|---|
| ATQ 141 | 4 | Lama1, Gm436, Phf20, Vmn2r45 |
| ATQ 270 | 3 | Plscr4, Chd5, Ripk2 |
| ATQ 274 | 3 | Mtap1 a, Csf2ra, G2e3 |
| ATQ 281 | 16 | Zfp457 (2)*, Grin2b, Ppp3cc, Olfr968, Trf, Sbf2, Chpt1, Enpp1, Stx4a (4), Ikzf4, Slc16a5, Fam71b |
| ATQ 290 | 14 | Ivns1abp, Gykl1, D4Ertd22e, Tsen54, Pdia3, Tsc2, Glyr1, Trio, Skint6, Oprk1, Prrc2a, Duoxa1, 2410089E03Rik, Aim1 |
| ATQ 303 | 18 | Ret, Zfp369, Olfr1006, Trip12, Pot1a, Vmn2r51, Wdr69, Larp1 b, Myh15, Serpine1, Cltc, Creld2, Olfr1462, 2610507B11 Rik (2), Ptar1, Adamts9, Trim28 |
| ATQ 799 | 50 | Lars, Serbp1, B3gat1, Usp37, Hacl1, Apol6, Oxr1, Cpd, Zc3h12a, Flt4, Trim41, Ireb2, Pgm1, Vwa2, Bhlhe40, Fer1l4, Thpo, Ict1, Mll2, Cmpk2, Alg10b, Pik3ca, Vmn1r191, Bmp5, Dock6, Acy3, Gria4, Brip1, H2-M10.3, Lce1e, BC055004, Gpr50, Mamdc2, Reep6, Tas2r122, Hectd1, Cep164, Otop3, Olfr733, Prss54, 2410089E03Rik, Micalcl, Rrm1, Itga11, Kirrel2, Olfr617, Nras, Dpf1, Rin2, Actb |
| BEH 112 | 2 | Dub1, Zfyve9 |
| BEH 280 | 7 | Skint6, Plxnd1, Hsp90aa1, Prcp, Cytl1, Amdhd1, Olfr1395 |
| BEH 420 | 13 | Tep1, Actc1, Psmb7, Loxl4, Krt36, Ccng1, Scarf1, Fat3, Radil, Tcfap2a, Vmn2r77 (2), Scn1a |
| BEH 461 | 20 | Sytl3, Pigb, Golgb1, Nipbl, Olfr895, Exosc10, Mfsd4, Igf1r, Col16a1, Trip12, Derl2, Zfp423, Rgma (3), Zfp759 (2), Vmn2r67(2), Nbn |

| | | |
|---|---|---|
| * Numbers in parentheses indicate distinct mutations appearing within the corresponding gene. | | |

### A novel K-Ras/Tp53 driven PDAC tumor model for the evaluation of the antitumor and toxic effects of potential therapeutic targets

Target ablation at the time of tumor initiation does not reflect therapeutic intervention in the clinic. Moreover, in most studies, targets are selectively ablated either in selected tissues or in those cells that express the oncogenic insult(s)^{24,25}. These strategies fail to provide relevant information regarding the potential toxic effects that might occur in the clinic where the targets are inhibited systemically. To address these issues, we developed a new GEM strain, K-*Ras*^{+/FSFG12V};*Tp53*^{frt/frt};*Elas*-tTA/*TetO*-Flp(o);Tg.h*UBC-*CreERT2 (designated as KPeFC) that uses two distinct recombinases, Flp(o) and CreERT2. responsible for tumor induction and target ablation, respectively. Flp(o) expression is mediated by the same Tet-Off system used in KPeC mice. KPeFC animals develop PanlN lesions and PDAC tumors with complete penetrance and kinetics similar to those observed in the KPeC strain (Extended Data Fig. 1). Expression of the tamoxifen (TMX)-inducible CreERT2 recombinase is driven by the human Ubiquitin promoter, a locus expressed in all adult tissues²⁶. Thus, exposure of KPeFC mice to a TMX containing diet allows the systemic recombination of any conditional floxed alleles added to this strain.

### Systemic ablation of c-Raf and Egfr expression in adult mice induces tolerable toxicities

Many therapies fail in the clinic due to their unacceptable toxic effects. Thus, we examined those toxicities derived from systemic ablation of c-Raf and Egfr expression. by exposing 12 week old Tg.h*UBC*-CreERT2;c-*Raf*^{lox/lox};*Egfr*^{lox/lox} mice to a TMX containing diet for 15 weeks (n=5 males, n=5 females). This treatment resulted in the complete recombination of both floxed alleles (Fig. 2a). Tg.h*UBC*-CreERT2;c-*Raf*^{+/+};*Egfr*^{+/+} siblings were used as controls Although mice lost weight during the first three weeks of treatment, they mostly recovered a few weeks later (Fig. 2b). Tg.hUBC-CreERT2;c-*Raf*^{lox/lox};*Egfr*^{lox/lox} mice developed skin alterations such as hyperplasia and disorganization of the epidermis, hyperkeratosis, folliculitis and inflammation with increased numbers of mast cells and significant hair loss (Fig. 2c). Moreover, these animals occasionally developed ulcers and scabs (Fig. 2c). These toxic effects were also observed in mice carrying *Egfr*^{lox} but not c-*Raf*^{lox} alleles. Thus, indicating that they were mediated by loss of Egfr expression. These skin defects are highly reminiscent of the acneiform rash and folliculitis observed in human patients treated with EGFR inhibitors²⁷. In addition, their small intestine displayed a slight disorganization of the crypts with increased number of apoptotic cells, albeit the overall architecture and functionality of the tissue was not affected (Fig. 2d). This defect was also observed in Tg.h*UBC*-CreERT2 mice carrying individual *Egfr*^{lox} and c-*Raf*^{lox} alleles These observations, taken together, suggest that combined inhibition of c-Raf and EGFR signaling might be well tolerated by patients.

### Systemic ablation of c-Raf and Egfr induces regression of advanced PDAC tumors.

Next, we assessed the consequences of systemically ablating c-Raf and Egfr expression in mice carrying advanced PDAC tumors. Tumor bearing KPeFC (n=10) and KPeFC*;*c-*Raf*^{lox/lox};*Egfr*^{lox/lox} mice (n=45) carrying lesions ranging from 2 to 50 mm³ were exposed to a TMX-containing diet. Control KPeFC mice (n=10) died between 2 to 8 weeks following TMX exposure (Fig. 3a). In the KPeFC;c-*Raf*^{lox/lox};*Egfr*^{lox/lox} cohort, a significant percentage of mice (33 out of 45, 73%) had to be eliminated from the study due to inefficient Cre-mediated recombination (n=19) or the appearance of sarcomas and/or papillomas likely to result from spurious expression of the Flp(o) recombinase (n=14). Therefore, only 12 of the initial 45 mice (27%) could be evaluated in this trial.

Eight mice displayed a rapid decrease in tumor volume even after one week of TMX exposure. However, tumors present in two animals, designated as "Resistant" (RT) mice, started to grow rapidly after 6 to 8 weeks of TMX exposure resulting in their dead 4 to 5 weeks later (Extended Data Fig. 2a). Western blot analysis of tumor tissue revealed the absence of c-Raf and Egfr expression (Extended Data Fig. 2b). The tumor present in the "Resistant" RT2 mouse had a sarcomatoid phenotype as illustrated by the lack of expression of CK19 and pErk (Extended Data Fig. 2a).

The remaining mice, designated as "Responders" (R), became tumor-free by micro-ultrasound analysis within six weeks of TMX exposure (Fig. 3b, c). Four "Responder" mice (R1-R4) were sacrificed after six weeks of TMX exposure whereas the remaining animals, R5 and R6, were allowed to survive for 10 additional weeks. No tumor reappearance, at least by micro-ultrasound analysis, was observed during this time period (Fig. 3b). Detailed histological examination of their pancreata revealed normal tissue architecture (Fig. 3d). One mouse (R2) did not display any lesions at the location where the tumor was formerly located. Yet, the other "Responder" mice (R1, R3 and R4) exhibited a single scar lesion, presumably a remnant of their original tumor (Fig. 3d). These scars appeared as very small fibrotic lesions measuring between 0.05 mm³ to 0.5 mm³, thus, reflecting a reduction of tumor volume of more than 5,000-fold (Fig. 3d). They were mostly composed of a dense network of organized collagen fibers, along with a significant content of hyaluronic acid (Extended Data Fig. 3). We also observed signs of chronic inflammation characterized by the presence of macrophages and T lymphocytes at their edges (Extended Data Fig. 3). Moreover, they contained few CK19 positive cells that were organized as ductal structures (Fig. 3d, e). They showed low levels of pErk staining as well as limited proliferative activity as illustrated by the presence of few Ki67 positive cells (Fig. 3f). Few cells displayed atypia and loss of cellular architecture, suggesting that they could represent residual neoplastic cells. Indeed, some epithelial cells in those lesions present in R1 and R6 mice retained Egfr and pErk expression, suggesting that they may represent residual unrecombined tumor cells (Fig. 3f).

The pancreata of these "Responder" mice contained occasional low grade PanlNs, (3 to 10 per mouse) including the R2 mouse in which the original PDAC tumor had completely disappeared. Most of these lesions expressed Egfr (Extended Data Fig. 4). Whether these PanlNs are derived from cells that were not able to progress to malignant lesions or represent late events during the course of the study remains to be determined.

Finally, four KPeFC;c-*Raf*^{lox/lox};*Egfr*^{lox/lox} mice did not respond to the TMX diet. Tumors present in these animals, designated as "Non Responders" (NR), progressed similarly to those present in control KPeFC mice (Fig. 4a). Histopathological analysis did not reveal significant differences with tumors present in control animals or in KPeFC;c-*Raf*^{lox/lox};*Egf*r^{lox/lox} mice not exposed to the TMX diet (Fig. 4c). As expected, these tumors did not express c-Raf or Egfr, yet they retained active MAPK and PI3K/Akt signaling pathways (Fig. 4c). Therefore, these tumors must have undergone additional alterations that made them independent of c-Raf and Egfr signaling. Alternatively, they may have originated from a putative distinct type of acinar cell.

### Transcriptional differences between pancreatic tumor cells sensitive and resistant to combined c-Raf and Egfr ablation

To gain insights on the potential mechanisms responsible for the lack of response to c-Raf and Egfr ablation in NR tumors, we generated cell lines from six tumors obtained from KPeFC*;*c-*Raf*^{lox/lox};*Egfr*^{lox/lox} mice. Ablation of c-Raf and Egfr expression upon infection with Ad-Cre particles led to complete inhibition of proliferation in three cells lines (designated as "Responder" cells, RC). In contrast, proliferation of the other three was unaffected ("Non Responder" cells, NRC) (Fig. 5a, b). Comparative analysis of the transcriptional profiles of these cell lines revealed distinct patterns of expression (Fig. 5c). RC cells were mainly characterized by higher levels of expression of CK19 and Gata6, two markers differentially expressed in the original tumors used to generate the RC cells lines (Extended Data Fig. 5). Moreover, Gene Set Enrichment Analysis (GSEA) identified several pathways enriched in NRC versus RC cells (Fig. 5d). The most differentially upregulated gene sets were those corresponding to the "E2F targets", "EMT" and "Myc targets". Other upregulated pathways included the "PI3K/Akt/Mtor" and "IL6/Jak/Sat3" signaling pathways. Differentially downregulated gene sets included those corresponding to "fatty acid and cholesterol metabolism" as well as "apoptosis" and the "p53 pathway". (Fig. 5d). Comparison of data obtained by RNA sequencing (RNAseq) analysis with a transcriptional classification of human PDAC tumors reported by Bailey and cols.²⁸, revealed that NRC cells displayed a transcriptional profile most similar to the "squamous cell type". In contrast, RC cells fit best with the other three classifications, "Immunogenic", "ADEX" and "Progenitor"²⁸ (Fig. 5e).

Subsequent analysis of known K-Ras effectors in NRC and RC cells failed to demonstrate significant differences in the phosphorylation levels of the Erk and Akt kinases as well as in pCofilin (Fig. 5f). Thus, suggesting that the MAPK, PI3K and Rock1 pathways might not be responsible for the proliferation of NRC cells in the absence of c-Raf and Egfr expression. Interestingly, ablation of *c-Raf* and *Egfr* in NRC cells induced increased phosphorylation levels of Stat3 at the canonical Tyr705 residue (Fig. 5f). These observations were further substantiated by IHC analysis (Fig. 5g, h). Tumors present in "Non Responder" mice as well as in mice that became resistant to c-*Raf* and *Egfr* ablation, constitutively expressed high levels of nuclear pStat3 after elimination of c-Raf and Egfr expression (Fig. 5g, h). No increase in pStat3 expression was identified in tumors of control KPeFC;c-*Raf*^{+/+};*Egfr*^{+/+} mice nor in those tumors cells present in the residual scars of "Responder" mice (Fig. 5g, h).

### c-RAF and EGFR are essential for proliferation of patient-derived pancreatic tumors.

To determine whether c-RAF and EGFR inhibition could provide therapeutic benefit to human PDAC tumors, we knocked down their expression in four human PDAC cell lines generated from patient derived xenograft (PDX) tumor models harboring *KRAS* and *TP53* mutations (Extended Data Fig. 6). Combined down regulation of c-RAF or EGFR expression completely interfered with the proliferative capacity of all four PDX cell lines (Extended Data Fig. 6). Finally, we injected these cells shRNA expressing PDAC tumor cells in immunocompromised mice (n=4). As illustrated in Fig. 6, combined downregulation of c-RAF and EGFR expression also compromised growth of these PDAC tumor cells *in vivo.* These observations indicate that human PDAC tumors cells also require c-RAF and EGFR expression for proliferation.

### Mice.

*Elas*-tTA/*Tet*O-Cre*;*K-*Ras*^{+/LSLG12Vgeo}, *Tp53*^{lox/lox}, *Egfr*^{lox/lox}, c-*Raf*^{lox/lox}, K-*Ras*^{+/FSFG12V}, *Tp53*^{frt/frt} and Tg.h*UBC*-CreERT2^{+/T} strains have been previously described^{16, 26, 37-41}. The transgenic *Tet*O-FLp(o) strain was generated by pronuclear injection of CMV-*Tet*O-FLp(o) DNA into B6.CBA zygotes⁴². All mice were maintained in a mixed 129/Sv-C57BL/6 background. Immunodeficient NU*-Foxn1nu* mice (females, 5-weeks-old) were purchased from Harlan Laboratories. All animal experiments were approved by the Ethical Committees of the Spanish National Cancer Research Centre (CNIO) and they were performed in accordance with the guidelines stated in the International Guiding Principles for Biomedical Research Involving Animal, developed by the Council for International Organizations of Medical Sciences (CIOMS). All strains were genotyped by Transnetyx (Cordova, Tennessee, USA).

### Tumor monitorization.

Mice were anesthetized with a continuous flow of 1% to 3% isoflurane in 100% oxygen at a rate of 1.5 liter/min. Hypothermia associated with anesthesia was avoided using a bed-heater. Abdominal hair was removed by depilation cream to prepare the examination area. Tumors were measured with a micro-ultrasound system (Vevo 770, Visualsonics) with an ultrasound transducer of 40 MHz (RMV704, Visualsonics). PDAC size was calculated as Length x Width²/2.

### Tamoxifen exposure.

Activation of the inducible CreERT2 recombinase encoded by the Tg.h*UBC*-CreERT2 transgene was achieved by feeding the mice with a tamoxifen-containing diet (Teklad CRD TAM400 diet, Harlan) *ad libitum.*

### Histopathology and immunohistochemistry.

For routine histological analysis, specimens were fixed in 10% buffered formalin (Sigma) and embedded in paraffin. For histopathological analysis, tissues were serially sectioned (3µm thick) and stained by conventional H&E every ten sections. Antibodies used for immunostaining included those raised against: CK-19 (CNIO Monoclonal Antibodies Core Unit), cleaved Caspase-3 (Cell Signaling Technology; 9661), CD3 (Santa Cruz Biotechnology, M-20), Egfr (Abcam, ab52894), pErk (Cell Signaling Technology; 9101), F4/80 (ABD Serotec, CI: A3-1), Gata6 (R&D Systems, AF1700), Ki67 (Master Diagnostica, 0003110QD), HABP (Millipore, 385911) and pStat3 (Cell Signaling Technology; 9145). Stained slides were scanned using the Mirax scanner (Zeiss). Images were analyzed by Zen2 software and photos were exported using Zen 2 software (Zeiss).

### X-Gal staining, Laser Capture Microdissection and PCR analysis.

X-Gal staining, laser capture microdissection and Egfr PCR analysis have been previously described¹². *c-Raf* wild type, floxed and null alleles were identified with forward c*-Raf* 1F (5'-CTGATTGCCCAACTGCCATAA-3'), c*-Raf* 3F (5'-GAGTCAGCAAATGCACTGAAATG-3') and reverse c*-Raf* 1R (5'-ACTGATCTGGAGCACAGCAAT-3') primers at 94°C for 1 minute, followed by 35 cycles of denaturation at 94°C for 30 seconds, annealing at 60°C for 30 seconds and extension at 72°C for 30s, and finally, followed by a long extension at 72°C for 10 minutes. These primers yielded DNA products of 196bp, 270bp and 143bp for wild-type, floxed and null c-Raf alleles respectively.

### Mouse PDAC cell cultures.

To generate mouse PDAC explants, freshly isolated tumors were minced with sterile razor blades, digested with collagenase P (1.5µg/ml) in Hank's Balanced Salt Solution (HBSS) for 30 min at 37º C, and cultured in DMEM with 10% of fetal bovine serum (FBS) and 1% Penicillin/Streptomycin. All studies were done on cells maintained in culture for less than ten passages. Their corresponding genotypes were verified by PCR analysis. PDAC cells explants were infected with Adeno-Cre particles (multiplicity of infection, 100) and seeded for colony formation assay 5 days after. Adeno-GFP particles were used as negative controls. Cells were seeded in equal cell numbers (5×10³) and allowed to form colonies for 2 weeks. Plates were fixed with 0.1% glutaraldehyde (Sigma) and stained with 0.5% Crystal Violet (Merck). Colonies were counted and quantified.

### PDX tumor models.

PDX tumors models utilized in this study include Panc-1 and Panc-4 (B.S. Jr, unpublished) as well as PDAC003T y PDAC013T (ref. 43) cell lines. For knockdown assays, PDX cells were infected with lentiviral supernatants expressing shRNAs against EGFR (TRCN0000121206 and TRCN0000121203), c-RAF (TRCN0000001068 and TRCN0000001065). c-RAF plasmid TRCN0000001065 selection was changed to blasticidine. A scrambled shRNA control vector was used as a negative control. Infected PDX cell lines were seeded in 96-well plates at a density of 1,500 cells per well and proliferation was assessed using the MTT assay. Infected PDX cells (0.5x10⁶) were injected 1:1 in PBS:Matrigel Matrix (Corning, 354234) into dorsal flanks of the mice. Growth was measured every 3 days until humane end point. Tumor volume was limited to 1000 mm³. Tumors were measured by caliper and calculated as Length x Width²/2.

### Western blot analysis.

Protein extracts (25 µg) obtained from tumor tissue or cell lines were separated on SDS/PAGE gels (Thermo Fisher Scientific), transferred to a nitrocellulose membrane and blotted with antibodies raised against Egfr (Abcam, ab52894), c-Raf (BD Biosciences, 610151), Erk-1 (BD Biosciences 554100), Erk-2 (BD Biosciences, 610103), pErk1/2 (Cell Signaling, 9101), Akt (Cell Signaling, 9272), pAkt (Cell Signaling, 4060), Stat3 (Cell Signaling, 9132), pStat3 (Cell Signaling, 9131), pCofilin (Santa Cruz, sc-21867-R) and Gapdh (Sigma, G8795). Primary antibodies were detected against mouse or rabbit IgGs (HRP, Dako and Alexa Fluor 680, Invitrogen) and visualized with ECL Western blot detection solution (GE Healthcare) or Odyssey infrared imaging system (LI-COR Biosciences).

### NGS DNA sequencing.

Total DNA was isolated from mouse PDAC cell explants using the Qiagen Dneasy Blood and Tissue kit according to manufacturer's instructions. Exomic NGS was carried out at the Columbia Genome Center, Columbia University, NY.

### RNAseq and GSEA analysis.

RNA from PDAC cell explants was extracted with Qiagen RNeasy Mini Kit, 1 µg of total RNA was used for further analysis. PolyA+ fraction was purified and randomly fragmented, converted to double stranded cDNA and processed through subsequent enzymatic treatments of end-repair, dA-tailing, and ligation to adapters as in Illumina's "TruSeq Stranded mRNA LT Sample Prep Kit" (this kit incorporates dUTP during 2nd strand cDNA synthesis, which implies that only the cDNA strand generated during 1st strand synthesis is eventually sequenced). The adapter-ligated library was completed by PCR with Illumina PE primers. The resulting purified cDNA library was applied to an Illumina flow cell for cluster generation and sequenced on an Illumina NovaSeq 6000 instrument by following manufacturer's guidelines. 101 bp single-end reads were analyzed with the Nextpresso pipeline⁴⁴ as follows: sequencing quality was verified with FastQC v0.11.0 (http://www.bioinformatics.babraham.ac.uk/projects/fastqc/). Reads were aligned to the mouse genome (NCBI37/mm9) with TopHat-2.0.10 (ref. 45) using Bowtie 1.0.0 (ref. 46) and SAMtools 0.1.19 (ref 47), allowing 2 mismatches and 20 multihits. Differential expression was tested with DESeq2 (ref 48) using the *Mus musculus* NCBI37/mm9 transcript annotations from https://ccb.jhu.edu/software/tophat/igenomes.shtml. GSEAPreranked⁴⁹ was used to perform a gene set enrichment analysis of the described gene signatures on a preranked gene list, setting 1000 gene set permutations. Gene Set Variation Analysis (GSVA)⁵⁰, was used to estimate the variation of pathway activity over the samples in an unsupervised manner. Heatmaps presented in this study were built with GENE-E software package (https:/:software.broadinstitute.org/GENE-E/index.html). RNA-seq data are available from the Gene Expression Omnibus (GEO) database under the following ID: GSE112434.

### Statistics and data analysis.

Data are represented as mean±s.d. Significance was calculated with the unpaired Student's t test using GraphPad Prism software. A p-value that was less than 0.05 was considered to be statistically significant for all data sets. Significant differences between experimental groups were: *p< 0.05, **p< 0.01 or ***p< 0.001.

### REFERENCES

1. Rahib, L. et al. Cancer Research 74, 2913-2921 (2014).
2. Hidalgo, M. N. Engl. J. Med. 362, 1605-1617 (2010).
3. Burris III, H. A. *et al.* **15,** 2403-2413 (1997).
4. Moore, M. J. et al. J. Clin. Oncol. 25, 1960-1966 (2007).
5. Von Hoff, D. D. et al. N. Engl. J. Med. 369, 1691-703 (2013).
6. Garrido-Laguna, I. & Hidalgo, M. Nat. Rev. Clin. Oncol. 12, 319-334 (2015).
7. Maitra, A. & Hruban, R. H. Annu. Rev. Pathol. 3, 157-88 (2008).
8. Ying, H. et al. Cell 149, 656-670 (2012).
9. Collins, M. A. et al. J. Clin. Invest. 122, 639-653 (2012).
10. Ostrem, J. M. et al. Nature 503, 548-551 (2013).
11. Cox, A. D. et al. Nat. Rev. Drug Discov. 13, 828-851 (2014).
12. Navas, C. et al. Cancer Cell 22, 318-30 (2012).
13. Ardito, C. M. et al. Cancer Cell 22, 304-317 (2012).
14. Sanclemente, M. et al. Cancer Cell 1-12 (2018). doi:10.1016/j.ccell.2017.12.014
15. Blasco, R. B. et al. Cancer Cell 19, 652-663 (2011).
16. Guerra, C. et al. Cancer Cell 11, 291-302 (2007).
17. Aguirre, A. J. et al. Genes Dev. 17, 3112-3126 (2003).
18. Bardeesy, N. et al. Proc. Natl. Acad. Sci. U. S. A. 103, 5947-5952 (2006).
19. Hingorani, S. R. et al. Cancer Cell 7, 469-483 (2005).
20. Guerra, C. et al. Cancer Cell 19, 728-739 (2011).
21. Hingorani, S. R. et al. Cancer Cell 4, 437-450 (2003).
22. Chung, W.-J. et al. Proc. Natl. Acad. Sci. U. S. A. 114, E10947-E10955 (2017).
23. Jones, S. et al. Science (80-.). 321, 1801-1806 (2008).
24. Drosten, M. et al. Cold Spring Harb. Perspect. Med. a031542 (2017). doi:10.1101/cshperspect.a031542
25. Pérez-Mancera, P. A. et al. Gastroenterology 142, 1079-1092 (2012).
26. Ruzankina, Y. et al. Cell Stem Cell 1, 113-26 (2007).
27. Owczarczyk-Saczonek, A. et al. Postep. Dermatologii i Alergol. 30, 195-198 (2013).
28. Bailey, P. et al. Nature 531, 47-52 (2016).
29. Kamerkar, S. et al. Nature 546, 498 (2017).
30. Todoric, J. et al. Cancer Cell 32, 824-839.e8 (2017).
31. Chio, I. I. C. et al. Cell 166, 963-976 (2016).
32. Moffitt, R. A. et al. Nat. Genet. 47, 1168-1178 (2015).
33. Collisson, E. A. et al. Nat. Med. 17, 500-503 (2011).
34. Mueller, S. et al. Nature 554, 62-68 (2018).
35. Raphael, B. J. et al. Cancer Cell 32, 185-203.e13 (2017).
36. Torres, C. & Grippo, P. J. Ann. Med. 1-11 (2018).
37. Jonkers, J. et al. Nat. Genet. 29, 418-25 (2001).
38. Natarajan, A. et al. Proc. Natl. Acad. Sci. U. S. A. 104, 17081-6 (2007).
39. Jesenberger, V. et al. J. Exp. Med. 193, 353-364 (2001).
40. Nieto, P. et al. Nature 548, 239-243 (2017).
41. Lee, C.-L. et al. Dis. Model. Mech. 5, 397-402 (2012).
42. Pease, S. & Saunders, T. L. (Springer, 2011).
43. Nicolle, R. et al. Cell Reports 21, 2458-2470 (2017).
44. Graña, O. et al. Curr. Bioinform. 12, (2017).
45. Trapnell, C. et al. Nat. Protoc. 7, 562-578 (2012).
46. Langmead, B. et al. Genome Biol. 10, R25 (2009).
47. Li, H. et al. Bioinformatics 25, 2078-9 (2009).
48. Love, M. I. et al. Genome Biol. 15, (2014).
49. Subramanian, A. et al. Proc. Natl. Acad. Sci. 102, 15545-15550 (2005).
50. Hänzelmann, S. et al. BMC Bioinformatics 14, 1-20 (2013).

## Claims

1. A pharmaceutical composition comprising an inhibitor of the expression, activity and/or function of c-Raf and an inhibitor of the expression, activity and/or function of EGF Receptor (EGFR).

2. The pharmaceutical composition according to the preceding claim, wherein the inhibitor of the expression, activity and/or function of c-Raf comprises a c-Raf inhibitor compound, a c-Raf inhibitor antibody or an antigen binding fragment thereof, a peptide or a nucleotide sequence.

3. The pharmaceutical composition according to any one of the preceding claims, wherein the inhibitor of the expression, activity and/or function of c-Raf does not inhibit C-RAF kinase activity.

4. The pharmaceutical composition according to any one of the preceding claims, wherein the inhibitor of the expression, activity and/or function of c-Raf is other than sorafenib, vemurafenib, dabrafenib y LY3009120.

5. The pharmaceutical composition according to any one of the preceding claims, wherein the inhibitor of the expression, activity and/or function of c-Raf does not inhibit the expression, activity and/or function of b-Raf.

6. The pharmaceutical composition according to any one of the preceding claims, wherein the inhibitor of the expression, activity and/or function of EGFR comprises an EGFR inhibitor compound, an EGFR inhibitor antibody or an antigen binding fragment thereof, a peptide or a nucleotide sequence.

7. The pharmaceutical composition according to any one of the preceding claims, wherein the nucleotide sequence is or codifies for a guide RNA, an interfering RNA or a micro RNA.

8. The pharmaceutical composition according to any one of the preceding claims, wherein the inhibitor of the expression, activity and/or function of EGFR is selected from afatinib, erlotinib, gefitinib, brigatinib, icotinib, neratinib, lapatinib, vandetanib, osimertinib, cetuximab, panitumumab, necitumumab, nimotuzumab, zalutumumab, matuzumab and combinations thereof.

9. The pharmaceutical composition according to any one of the preceding claims, comprising a therapeutically effective amount of an inhibitor of the expression, activity and/or function of c-Raf, a therapeutically effective amount of an inhibitor of the expression, activity and/or function of EGFR and optionally a pharmaceutically acceptable excipient.

10. The pharmaceutical composition according to any one of the preceding claims for use in the prevention and/or treatment of cancer.

11. The pharmaceutical composition according to any one of the preceding claims for use in the prevention and/or treatment of pancreatic cancer.

12. The pharmaceutical composition according to any one of the preceding claims for use in the prevention and/or treatment of pancreatic intraepithelial neoplasia or of pancreatic ductal adenocarcinoma.

13. The pharmaceutical composition according to any one of the preceding claims for use in tumor regression in a subject afflicted with cancer.

14. The pharmaceutical composition according to any one of the preceding claims for use in tumor regression in a subject afflicted with pancreatic cancer.

15. The pharmaceutical composition according to any one of the preceding claims for use in tumor regression in a subject afflicted with pancreatic intraepithelial neoplasia or of pancreatic ductal adenocarcinoma.
